# EUROPEAN PATENT APPLICATION

(11) **EP 4 721 800 A1**
(43) Date of publication of application: **08.04.2026**
(21) Application number: 24815736.4
(22) Date of filing: 13.05.2024
(51) Int. Cl.: A61M 21/00, A61N 1/36, A61N 1/04, A24F 47/00

(54) **WEARABLE DEVICE AND METHOD FOR PROVIDING SMOKING EFFECT**

(30) Priority: 26.05.2023 KR 20230068151; 16.06.2023 KR 20230077203; 01.08.2023 KR 20230100276
(71) Applicant: KT&G Corporation, Daedeok-gu Daejeon 34337 (KR)
(72) Inventor: LEE, Wonkyeong, Guri-si Gyeonggi-do 11920 (KR); KIM, Min Kyu, Seoul 08211 (KR)
(74) Representative: Ter Meer Steinmeister & Partner
(86) International application number: PCT/KR2024/006434
(87) International publication number: WO 2024/248358

(57) **Abstract**

This wearable device providing a smoking effect comprises: a housing; a first stimulation unit provided on one surface of the housing and including at least one electrode disposed at a position corresponding to a first part of a user to provide electrical stimulation; a second stimulation unit provided on one surface of the housing and including at least one electrode disposed at a position corresponding to a second part of the user to provide electrical stimulation; and a controller for controlling the first stimulation unit or the second stimulation unit, wherein the first stimulation unit or the second stimulation unit can provide an electrical stimulation signal for inducing a state of arousal in which a smoking effect can be generated.

## Description

### TECHNICAL FIELD

Methods and apparatuses consistent with embodiments relate to a wearable device and a method for providing a smoking effect.

### BACKGROUND ART

Research on an apparatus for generating electroencephalogram (EEG) is in progress. For example, Korean Patent Publication No. 10-2011-0064706 discloses an apparatus and method for generating EEG.

### DISCLOSURE OF THE INVENTION

### TECHNICAL GOALS

Embodiments provide a smoking effect to a user without an act of smoking.

Embodiments effectively provide a smoking effect through stimulation of the brain of a user.

Embodiments effectively provide a smoking effect by performing brain stimulation on a plurality of regions of the brain of a user.

Embodiments provide an effective control of the degree of brain stimulation based on feedback from a user.

One or more embodiments may address at least the above problems and/or disadvantages and other disadvantages not described above. Also, the embodiments are not required to overcome the disadvantages described above, and an embodiment may not overcome any of the problems described above.

### TECHNICAL SOLUTIONS

According to an aspect of an embodiment, there is provided a wearable device for providing a smoking effect. The wearable device includes a housing, a first stimulus unit provided on a surface of the housing and comprising at least one electrode that is disposed on a position corresponding to a first region of a user and configured to provide electrical stimulation, a second stimulus unit provided on the surface of the housing and comprising at least one electrode that is disposed on a position corresponding to a second region of the user and configured to provide electrical stimulation, and a controller configured to control the first stimulus unit or the second stimulus unit, wherein the first stimulus unit or the second stimulus unit is configured to provide an electrical stimulus signal that induces an alertness state capable of implementing a smoking effect.

The wearable device may further include a third stimulus unit provided on the surface of the housing, wherein the third stimulus unit may include at least one electrode that may be disposed on a position corresponding to a third region of the user and configured to provide electrical stimulation.

The first region may be a frontal lobe, the second region may be a parietal lobe, and the third region may be an occipital lobe.

The controller may further be configured to control at least one of the first stimulus unit, the second stimulus unit, or the third stimulus unit so that the first stimulus unit, the second stimulus unit, or the third stimulus unit may provide a brain stimulus signal that may decrease delta waves or theta waves of the user or increase beta waves of the user.

The controller may further be configured to activate the second stimulus unit or the third stimulus unit to decrease delta waves of the user.

The controller may further be configured to activate the second stimulus unit or the third stimulus unit, and the second stimulus unit or the third stimulus unit may be configured to provide electrical stimulation that may cause power spectral density (PSD) of electroencephalogram (EEG) of the user to decrease by 0.3 to 0.9 dB in a frequency range of 1 Hz to 4 Hz.

The controller may further be configured to activate the first stimulus unit or the third stimulus unit to decrease theta waves of the user.

The controller may further be configured to activate the first stimulus unit or the third stimulus unit, and the first stimulus unit or the third stimulus unit may be configured to provide electrical stimulation that may cause PSD of EEG of the user to decrease by 0.2 to 0.5 dB in a frequency range of 4 Hz to 8 Hz.

The controller may further be configured to activate at least one of the first stimulus unit, the second stimulus unit, or the third stimulus unit to increase beta waves of the user.

The controller may further be configured to activate at least one of the first stimulus unit, the second stimulus unit, or the third stimulus unit, and at least one of the first stimulus unit, the second stimulus unit, or the third stimulus unit may be configured to provide electrical stimulation that may cause PSD of EEG of the user to increase by 0.1 to 1.1 dB in a frequency range of 12 Hz to 25 Hz.

The housing may be configured to be worn on a head of the user.

According to an aspect of an embodiment, there is provided a method of providing a smoking effect. The method includes disposing electrodes on at least two of positions corresponding to a frontal lobe, a parietal lobe, and an occipital lobe of a user and providing an electrical stimulus signal to the electrodes so that delta waves or theta waves of electroencephalogram (EEG) of the user decrease or beta waves of the EEG of the user increase.

In the providing of the electrical stimulus signal to the electrodes, the electrical stimulus signal may be provided to electrodes disposed on a position corresponding to the parietal lobe or the occipital lobe, and accordingly, the delta waves may decrease.

In the providing of the electrical stimulus signal to the electrodes, the electrical stimulus signal may be provided to electrodes disposed on a position corresponding to the frontal lobe or the occipital lobe, and accordingly, the theta waves may decrease.

In the providing of the electrical stimulus signal to the electrodes, at least one of the frontal lobe, the parietal lobe, or the occipital lobe may be activated, and accordingly, the beta waves may increase.

According to an aspect of an embodiment, there is provided a wearable device for providing a smoking effect. The wearable device includes a housing, a stimulus unit provided on a surface of the housing and configured to provide a stimulus signal, and a controller configured to control the stimulus unit, wherein the controller may control the stimulus unit so that the stimulus unit may generate a stimulus signal that induces a state of alertness capable of implementing a smoking effect.

The stimulus unit may generate a stimulus signal that stimulates a brain of a user.

The stimulus signal may be a brain stimulus signal that decreases at least one of delta waves or theta waves or increase beta waves.

The controller may control the stimulus unit so that the stimulus unit may generate a brain stimulus signal that increases a frequency region of EEG of the user corresponding to 10 Hz or higher.

The controller may control the stimulus unit so that the stimulus unit may generate a brain stimulus signal that causes PSD of EEG of the user to decrease by 0.3 to 0.9 dB in a frequency range of 1 Hz to 4 Hz.

The controller may control the stimulus unit so that the stimulus unit may generate a brain stimulus signal that causes PSD of EEG of the user to decrease by 0.2 to 0.5 dB in a frequency range of 4 Hz to 8 Hz.

The controller may control the stimulus unit so that the stimulus unit may generate a brain stimulus signal that causes PSD of EEG of the user to increase by 0.1 to 1.1 dB in a frequency range of 12 Hz to 25 Hz.

The stimulus unit may include a plurality of electrodes that may perform electrical stimulation and a power supply connected to the plurality of electrodes. At least one of the plurality of electrodes may be disposed in a position corresponding to the dorsolateral prefrontal cortex of the user.

The wearable device may further include a verification unit that may verify a smoking effect.

The controller may determine a degree of activation of the stimulus unit based on a smoking indicator signal transmitted from the verification unit.

The verification unit may measure a heart rate variability (HRV).

According to an aspect of an embodiment, there is provided a system for providing a smoking effect. The system includes a stimulus unit that performs brain stimulation on a brain of a user and a controller that controls a stimulus signal of the stimulus unit, wherein the controller may control the stimulus unit so that the stimulus unit may decrease at least one of delta waves or theta waves of the user and increase beta waves of the user.

The stimulus unit may provide electrical stimulation to a position corresponding to the dorsolateral prefrontal cortex of a user.

The system may further include a verification member that may verify a smoking effect of the user, and the verification member may transmit a smoking indicator signal detected from the user to the controller.

The controller may determine a degree of activation of the stimulus unit based on the smoking indicator signal transmitted from the verification member.

Additional aspects of embodiments will be set forth in part in the description which follows and, in part, will be apparent from the description, or may be learned by practice of the disclosure.

### EFFECTS OF THE INVENTION

According to embodiments, a smoking effect may be provided to a user without an act of smoking.

According to embodiments, a smoking effect may effectively be provided through stimulation of the brain of a user.

According to embodiments, a smoking effect may effectively be provided by performing brain stimulation on a plurality of regions of the brain of a user.

According to embodiments, the degree of brain stimulation may effectively be controlled based on feedback from a user.

The effects of a wearable device and a method of providing a smoking effect are not limited to the above-mentioned effects, and other unmentioned effects may be clearly understood from the following description by one of ordinary skill in the art.

### BRIEF DESCRIPTION OF THE DRAWINGS

The above and/or other aspects will be more apparent by describing certain embodiments with reference to the accompanying drawings, in which:
FIG. 1 is a diagram illustrating a wearable device according to an embodiment;
FIG. 2 is a diagram illustrating an electrode arrangement of the wearable device, according to an embodiment;
FIG. 3 is a diagram illustrating a use state of the wearable device according to an embodiment;
FIG. 4 is a diagram illustrating electroencephalogram (EEG) of a user before smoking and after smoking;
FIGS. 5A and 5B are diagrams illustrating a region of brain stimulation for reducing delta waves;
FIGS. 6A and 6B are diagrams illustrating a region of brain stimulation for reducing theta waves;
FIGS. 7A and 7B are diagrams illustrating a region of brain stimulation for reducing beta waves;
FIG. 8 is a diagram illustrating EEG of a user before and after stimulation by a wearable device according to an embodiment;
FIGS. 9A to 9E are diagrams illustrating indicators of a heart rate variability (HRV) of a user before smoking and after smoking;
FIGS. 10A and 10B are diagrams illustrating an autonomic balance among HRV indicators of a user before and after stimulation by a wearable device, according to an embodiment;
FIG. 11 is a diagram illustrating a method of providing a smoking effect, according to an embodiment; and
FIG. 12 is a diagram illustrating a system for providing a smoking effect, according to an embodiment.

### BEST MODE FOR CARRYING OUT THE INVENTION

The terms used in the embodiments are selected from among common terms that are currently widely used, in consideration of their function in the embodiments. However, the terms may become different according to an intention of one of ordinary skill in the art, a precedent, or the advent of new technology. Also, in particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that when a certain part "includes" a certain component, the part does not exclude another component but may further include another component, unless the context clearly dictates otherwise. Also, terms such as "unit," "module," etc., as used in the specification may refer to a part for processing at least one function or operation and may be implemented as hardware, software, or a combination of hardware and software.

As used herein, an expression such as "at least one of" that precedes listed components modifies not each of the listed components but all the components. For example, expressions "at least one of a, b, or c" and "at least one of a, b, and c" should be construed as referring to "a," "b," "c," "a and b," "a and c," "b and c," or "a, b, and c."

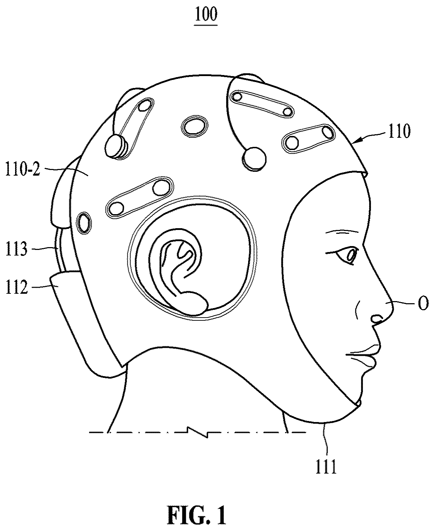

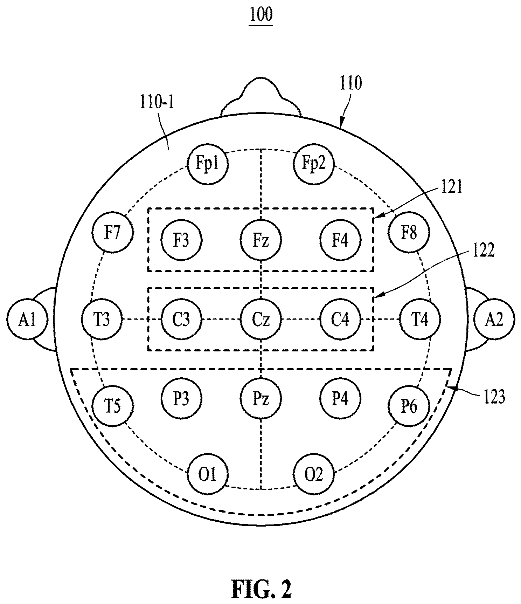

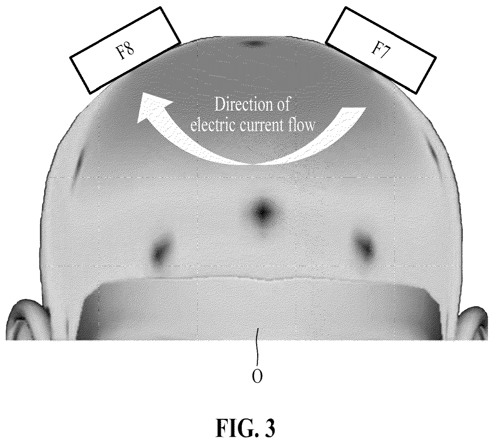

FIG. 1 is a diagram illustrating a wearable device 100 according to an embodiment, FIG. 2 is a diagram illustrating an electrode arrangement of the wearable device 100, according to an embodiment, and FIG. 3 is a diagram illustrating a use state of the wearable device 100 according to an embodiment.

Referring to FIGS. 1 to 3, the wearable device 100 may provide a smoking effect. The wearable device 100 may include a housing 110, a first stimulus unit 121 provided on a surface of the housing 110 and configured to provide a stimulus signal to a first region of a user, a second stimulus unit 122 provided on the surface of the housing 110 and configured to provide a stimulus signal to a second region of the user, and a controller configured to stimulate the first stimulus unit 121 or the second stimulus unit 122. The wearable device 100 may further include a third stimulus unit 123 configured to provide a stimulus signal to a third region of the user.

At least a portion of the housing 110 may be worn in contact with a body part of a user O. The housing 110 may be configured in a form that may be worn on the head of the user O, such as a helmet, a hat, and the like. For example, the housing 110 may be made of metal or plastic material. A portion (e.g., an edge) of the housing 110 may be made of metal material, and the other portion of the housing 110 may be made of plastic material.

The housing 110 may be securely worn on the head of the user O through a wearing means 111. For example, the wearing means 111 may have a form of a band or strap that may connect one end portion of the housing 110 to the other end portion of the housing 110 and may be composed of various materials (e.g., rubber material, plastic, and metal). The wearing means 111 may be configured to be detachable from the housing 110 according to the preference of the user O so that the appearance of the wearable device 100 may be given various aesthetics. In another example, the housing 110 may be worn in a forced manner on the head of the user O, without the wearing means 111.

The housing 110 may include a first surface 110-1 that contacts the user O, a second surface 110-2 opposite to the first surface 110-1, and a side surface between the first surface 110-1 and the second surface 110-2. The first stimulus unit 121, the second stimulus unit 122, and the third stimulus unit 123 may be disposed in an internal space of the housing 110, which may be defined by the first surface 110-1, the second surface 110-2, and the side surface. At least a portion of each of the first stimulus unit 121, the second stimulus unit 122, and the third stimulus unit 123 may be exposed outside the first surface 110-1. A memory, a communication unit, a power supply, and the like may additionally be accommodated in the internal space of the housing 110.

For example, the memory may be hardware for storing various pieces of data that are processed in the wearable device 100 and may store data that has been processed by the controller and data to be processed by the controller. The memory may include at least one type of storage medium of flash memory-type memory, hard disk-type memory, multimedia card micro-type memory, card-type memory (e.g., a secure digital (SD) or extreme digital (XD) memory), random-access memory (RAM), static random-access memory (SRAM), read-only memory (ROM), electrically erasable programmable read-only memory (EEPROM), programmable read-only memory (PROM), magnetic memory, a magnetic disk, or an optical disk. The memory may store an operating time, a maximum use time, and a current use time of the wearable device 100, and data on a smoking pattern of the user.

For example, the communication unit may include a short-range wireless communication unit and a wireless communication unit. The short-range wireless communication unit may include a Bluetooth communication unit, a Bluetooth Low Energy (BLE) communication unit, a near field communication unit, a wireless local area network (WLAN) (e.g., wireless fidelity (Wi-Fi)) communication unit, a ZigBee communication unit, an infrared data association (IrDA) communication unit, a Wi-Fi direct (WFD) communication unit, an ultra-wideband (UWB) communication unit, an Ant+ communication unit, and the like. However, embodiments are not limited thereto. The wireless communication unit may include a cellular network communication unit, an Internet communication unit, a computer network (e.g., a local area network (LAN) or a wide-area network (WAN)) communication unit, and the like. However, embodiments are not limited thereto.

A power supply or a stimulus generator 112 may be disposed on one side of the housing 110. The power supply or the stimulus generator 112 may be connected to the first stimulus unit 121, the second stimulus unit 122, and the third stimulus unit 123 via a connection cable 113. The power supply or the stimulus generator 112 may provide power or an electrical stimulus signal for brain stimulation to the first stimulus unit 121, the second stimulus unit 122, and the third stimulus unit 123.

In another example, the power supply or the stimulus generator 112 may be disposed spaced apart from the housing 110. For example, the power supply or the stimulus generator 112 may be configured in a form of a neckband and worn in a state of hanging around a neck of the user O. In this case also, the power supply or the stimulus generator 112 may be connected to the first stimulus unit 121, the second stimulus unit 122, and/or the third stimulus unit 123 via the connection cable 113.

The controller of the wearable device 100 may control the first stimulus unit 121, the second stimulus unit 122, and/or the third stimulus unit 123 so that the first stimulus unit 121, the second stimulus unit 122, and/or the third stimulus unit 123 may generate an electrical stimulus signal that induces a state of alertness that may implement a smoking effect. For example, the controller may be housed in the internal space of the housing 110 and connected to the first stimulus unit 121, the second stimulus unit 122, and/or the third stimulus unit 123. In another example, the controller may be accommodated in another electronic device (e.g., a mobile communication terminal) outside of the housing 110 and may provide a control signal to the first stimulus unit 121, the second stimulus unit 122, and/or the third stimulus unit 123 through the communication unit.

The first stimulus unit 121, the second stimulus unit 122, and/or the third stimulus unit 123 may include at least one electrode that performs electrical stimulation. The electrode may be connected to the controller. The electrode may be connected to the power supply or to the stimulus generator 112.

For example, a pad may be attached to a portion in which the electrode is exposed from the first surface 110-1 of the housing 110. The pad may be in contact with a scalp of the user O. The pad may be made of a hydrogel material to be used multiple times. The pad may be replaced after the end of its life.

In particular, referring to FIG. 2, the electrode may consist of a plurality of electrodes A1, A2, C3, C4, Cz, F3, F4, F7, F8, Fp1, Fp2, Fz, O1, O2, P3, P4, Pz, T3, T4, T5, and T6.

The first stimulus unit 121 may include a portion of the electrodes (e.g., F3, Fz, and F4) that may be disposed in a position corresponding to the first region (e.g., a frontal lobe) of the user O.

The second stimulus unit 122 may include a portion of the electrodes (e.g., C3, Cz, and C4) that may be disposed in a position corresponding to the second region (e.g., a parietal lobe) of the user O.

The third stimulus unit 123 may include a portion of the electrodes (e.g., T5, P3, Pz, P4, P6, O1, and O2) that may be disposed in a position corresponding to the third region (e.g., an occipital lobe) of the user O.

The controller of the wearable device 100 may change a state of the user O to an alertness state by providing an electrical stimulus signal to the first stimulus unit 121, the second stimulus unit 122, and/or the third stimulus unit 123. For example, referring to FIG. 3, the controller of the wearable device 100 may perform electrical stimulation to the dorsolateral prefrontal cortex of the user O by providing an electrical stimulus signal to the electrode F7 or the electrode F8 disposed in a position corresponding to the dorsolateral prefrontal cortex of the user O. The electrode F7 may consist of an anode (+), the electrode F8 may consist of a cathode (-), and the controller of the wearable device 100 may allow an electric current from the electrode F7 to flow through at least a portion of the dorsolateral prefrontal cortex of the user O to the electrode F8. Here, an excitability of neurons in the dorsolateral prefrontal cortex adjacent to the electrode F7 may increase and an excitability of neurons in the dorsolateral prefrontal cortex adjacent to the electrode F8 may be reduced so that an alertness effect of the user O may be maximized.

The controller of the wearable device 100 may control the first stimulus unit 121, the second stimulus unit 122, and/or the third stimulus unit 123 so that the first stimulus unit 121, the second stimulus unit 122, and/or the third stimulus unit 123 may generate a brain stimulus signal that induces a state of alertness that may implement a smoking effect.

The brain stimulus signal may be electrical stimulation. The brain stimulus signal may be transcranial alternating current stimulation (tACS), and the tACS may affect a change in periodic excitability of a neuron in a cerebral cortex with a maximum current within 2 mA of a sine waveform at various frequencies and may induce a change in a brain function such as cognition and memory.

The controller of the wearable device 100 may control the first stimulus unit 121, the second stimulus unit 122, and/or the third stimulus unit 123 so that the first stimulus unit 121, the second stimulus unit 122, and/or the third stimulus unit 123 may provide a brain stimulus signal that decreases at least one of a delta waves region or a theta waves region of electroencephalogram (EEG) of the user O or increases a beta waves region of the EEG of the user O.

A frequency range of delta waves may be from 1 Hz to 4 Hz, and delta waves are associated with an involuntary physical activity, such as a heartbeat and digestion. When power associated with delta waves is increased, a state of rest and sleep may be induced in a user O and a situation such as relaxation may be implemented. On the contrary, when the power associated with delta waves is decreased, a state of alertness may be induced. A frequency range of theta waves may be from 4 Hz to 8 Hz, and theta waves may mainly occur during meditation or when a hippocampus is activated. The hippocampus may be a region that controls memory. When power associated with theta waves is increased, a state of inspiration, insight, or connecting pieces of memory may be induced. On the contrary, when the power associated with theta waves is decreased, a state of anxiety, stress, and low emotional self-awareness may be induced. A frequency range of beta waves may be from 12 Hz to 25 Hz, and since beta waves normally occur during alertness, an increase in beta waves may indicate alertness. When power associated with beta waves is increased, a state of alertness such as a situation of cognitive thought activity may be induced. On the contrary, when the power associated with beta waves is decreased, a state of relaxation may be induced.

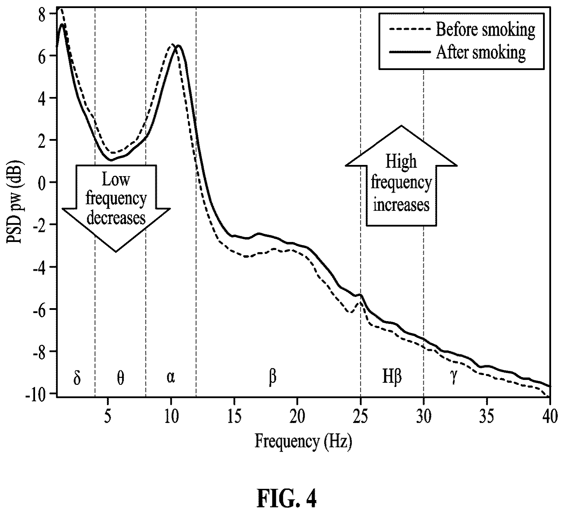

FIG. 4 is a diagram illustrating EEG of a user before and after smoking a cigarette or an electronic cigarette (e-cigarette).

The graph in FIG. 4 shows a measurement of the EEG of a subject before and after smoking a cigarette or an e-cigarette, and an electrophysiological method of recording an electrical activity of a brain through an electrode was used. The detailed method of the experiment is as follows. EEG sensors (i.e., sensors for measuring EEG) were attached to a plurality of positions on the heads of subjects. The subjects were divided into two groups, a cigarette group and an e-cigarette group, and EEG was measured before and after smoking in each of the cases. Results of analysis of power spectral density (PSD) before and after smoking are shown in the graph of FIG. 4. Referring to FIG. 4, as a result of analysis of pieces of power of all bands, it is confirmed that the low-frequency (e.g., delta waves and theta waves) range tends to decrease and the high-frequency (e.g., beta waves) range tends to increase after smoking. For example, it is confirmed that PSD of the EEG of the user in a frequency range of 10 Hz or higher tends to increase in general.

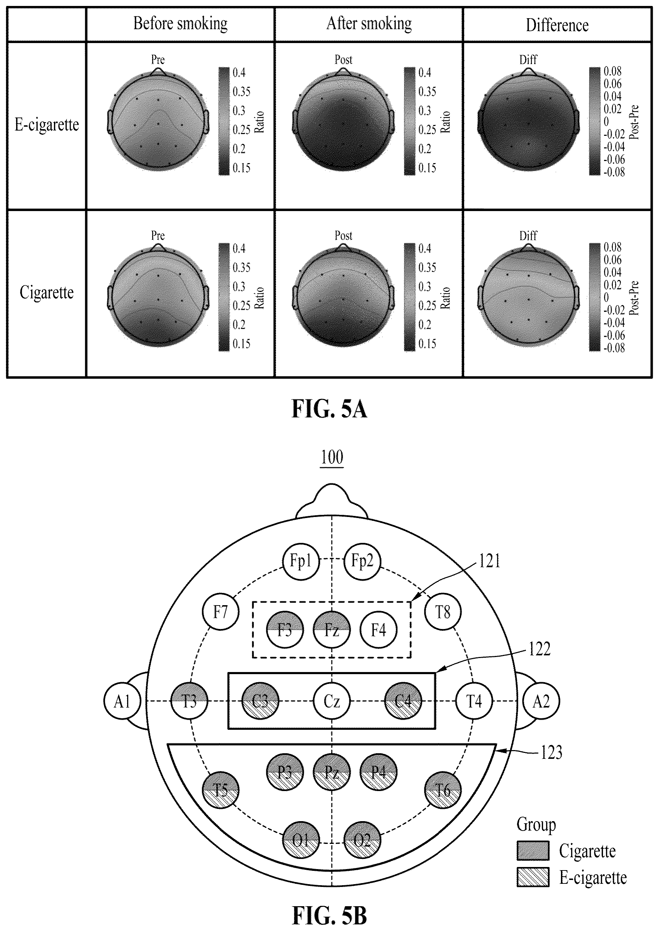

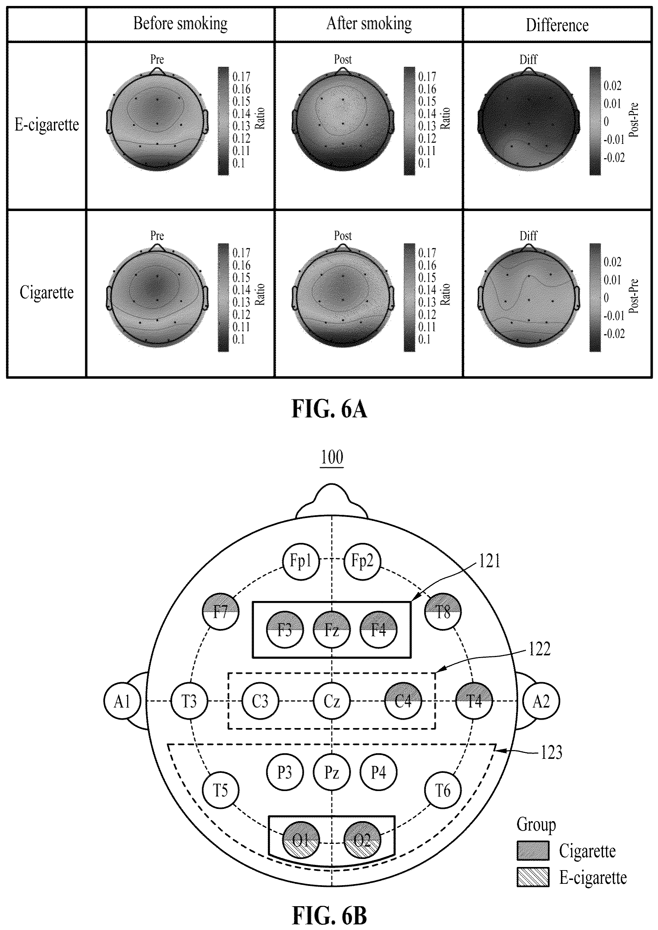

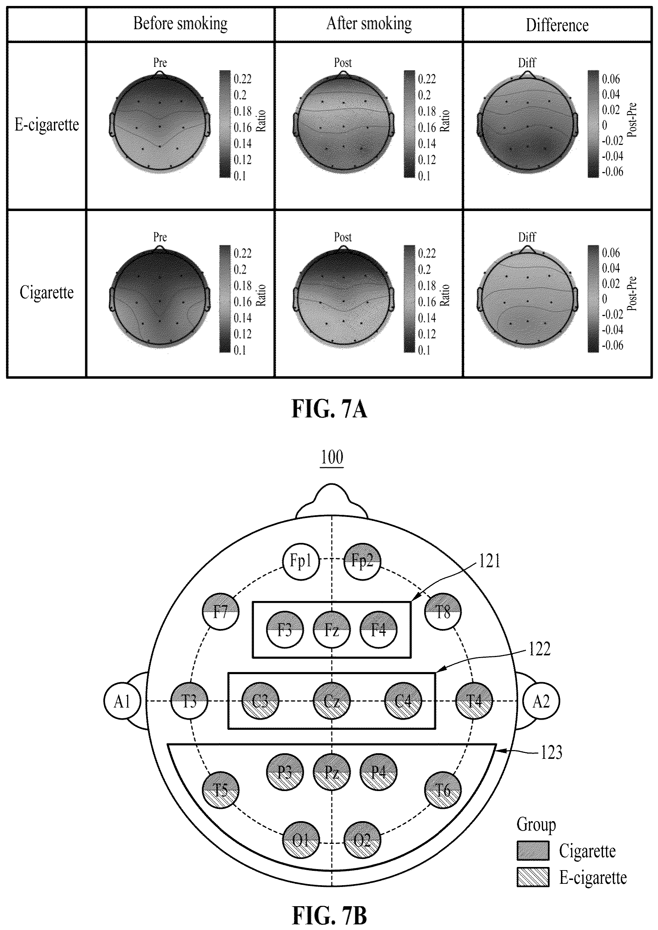

FIGS. 5A and 5B are diagrams illustrating a region of brain stimulation for reducing delta waves, and FIGS. 6A and 6B are diagrams illustrating a region of brain stimulation for reducing theta waves. FIGS. 7A and 7B are diagrams illustrating a region of brain stimulation for reducing beta waves.

FIG. 5A shows a pattern of EEG changes related to delta waves before and after smoking a cigarette and an e-cigarette, and FIG. 5B shows a region of brain stimulation of the user O for reducing delta waves.

Referring to FIG. 5A, with regard to delta waves, the occipital lobe and the parietal lobe may be activated by an e-cigarette, and the occipital lobe, the parietal lobe, and the frontal lobe (specifically, the dorsolateral prefrontal cortex) may be activated by a cigarette. Both a cigarette and an e-cigarette may activate regions of the occipital lobe and the parietal lobe.

Referring to FIG. 5B, a controller of the wearable device 100 may reduce delta waves by activating the second stimulus unit 122 and/or the third stimulus unit 123 to provide a smoking effect. The controller may provide electrical stimulation to the parietal lobe region and/or the occipital lobe region of the user O by activating the electrodes C3, Cz, and C4 of the second stimulus unit 122 and/or the electrodes T5, P3, Pz, P4, T6, O1, and O2 of the third stimulus unit 123 so that the delta waves of the user O may effectively be reduced.

For example, the controller of the wearable device 100 may activate the second stimulus unit 122 and/or the third stimulus unit 123, and the second stimulus unit 122 and/or the third stimulus unit 123 may provide electrical stimulation that causes PSD of EEG of the user O to decrease by 0.3 to 0.9 dB in a frequency range of 1 Hz to 4 Hz.

FIG. 6A shows a pattern of EEG changes related to theta waves before and after smoking a cigarette and an e-cigarette, and FIG. 6B shows a region of brain stimulation of the user O for reducing theta waves.

Referring to FIG. 6A, with regard to theta waves, the occipital lobe may be activated by an e-cigarette, and the frontal lobe (specifically, the dorsolateral prefrontal cortex) may be activated by a cigarette.

Referring to FIG. 6B, a controller of the wearable device 100 may reduce theta waves of the user O by activating the first stimulus unit 121 and/or the third stimulus unit 123. Here, the electrodes F3, Fz, and F4, among the plurality of electrodes of the first stimulus unit 121, in a position corresponding to the frontal lobe (specifically, the dorsolateral prefrontal cortex) may be activated. The controller may provide electrical stimulation to a frontal lobe region (specifically, a dorsolateral prefrontal cortex region) and/or an occipital lobe region of the user O by activating the electrodes F3, Fz, and F4 of the first stimulus unit 121 and/or the electrodes O1 and O2 of the third stimulus unit 123 so that the theta waves of the user O may effectively be reduced.

For example, the controller may activate the first stimulus unit 121 and/or the third stimulus unit 123, and the first stimulus unit 121 and/or the third stimulus unit 123 may provide electrical stimulation that causes PSD of EEG of the user O to decrease by 0.2 to 0.5 dB in a frequency range of 4 Hz to 8 Hz.

FIG. 7A shows a pattern of EEG changes related to beta waves before and after smoking a cigarette and an e-cigarette, and FIG. 7B shows a region of brain stimulation of the user O for increasing beta waves.

Referring to FIG. 7A, with regard to beta waves, the occipital lobe and the parietal lobe may be activated by an e-cigarette, and the occipital lobe, the parietal lobe, and the frontal lobe may be activated by a cigarette.

Referring to FIG. 7B, the controller may increase beta waves of the user O by activating the first stimulus unit 121, the second stimulus unit 122, and/or the third stimulus unit 123. The controller may provide electrical stimulation to a frontal lobe region, a parietal lobe region, and/or an occipital lobe region by activating the electrodes F3, Fz, and F4 of the first stimulus unit 121, the electrodes C3, Cz, and C4 of the second stimulus unit 122, and/or the electrodes T5, P3, Pz, P4, T6, O1, and O2 of the third stimulus unit 123 so that beta waves of the user O may effectively be increased.

For example, the controller may activate the first stimulus unit 121, the second stimulus unit 122, and/or the third stimulus unit 123, and the first stimulus unit 121, the second stimulus unit 122, and/or the third stimulus unit 123 may provide electrical stimulation that causes PSD of EEG of the user O to increase by 0.1 to 1.1 dB in a frequency range of 12 Hz to 25 Hz.

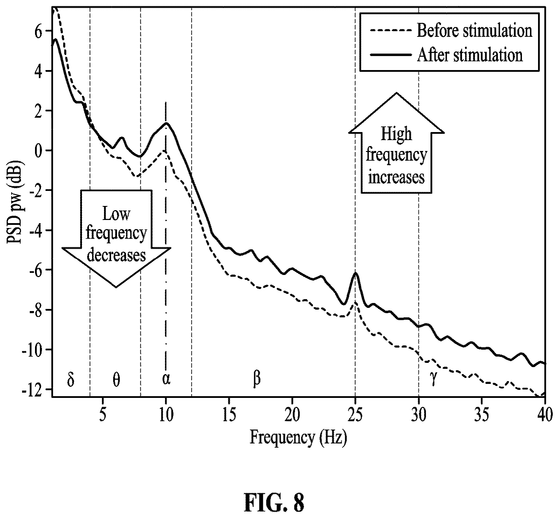

Referring to FIG. 8, a same/similar result as actual smoking shown in FIG. 4 was implemented using the wearable device 100.

With regard to a decrease of delta waves, which correspond to a low-frequency region, electrical stimulation may be provided to the parietal lobe and/or the occipital lobe of the user O by activating the second stimulus unit 122 and/or the third stimulus unit 123 of the wearable device 100. Through this, delta waves may effectively be decreased, thereby maximizing alertness of the user O, which is associated with providing a smoking effect.

With regard to a decrease of theta waves, which correspond to a low-frequency region, electrical stimulation may be provided to the dorsolateral prefrontal cortex and/or the occipital lobe of the user O by activating the first stimulus unit 121 and/or the third stimulus unit 123 of the wearable device 100. Through this, theta waves may effectively be decreased, thereby maximizing alertness of the user O, which is associated with providing a smoking effect.

With regard to an increase of beta waves, which correspond to a high-frequency region, electrical stimulation may be provided to all regions of the brain of the user O by activating the first stimulus unit 121, the second stimulus unit 122, and/or the third stimulus unit 123 of the wearable device 100. Through this, beta waves may effectively be increased, thereby maximizing alertness of the user O, which is associated with providing a smoking effect.

In addition, electrical stimulation from the first stimulus unit 121, the second stimulus unit 122, and/or the third stimulus unit 123 may be tACS, and the tACS may affect a change in periodic excitability of a neuron in a cerebral cortex with a maximum current within 2 mA of a sine waveform at various frequencies and may induce a change in a brain function such as cognition and memory. An applied electric current may be 1 to 3 mA, and a stimulation time may be 10 to 30 minutes.

In an embodiment, the wearable device 100 may control a stimulus unit (e.g., the first stimulus unit 121, the second stimulus unit 122, and the third stimulus unit) so that the stimulus unit may provide a brain stimulus signal that increases a frequency region of the brain of the user O corresponding to 10 Hz or higher.

In an embodiment, the wearable device 100 may control the stimulus unit so that the stimulus unit may provide first electrical stimulation that causes PSD of EEG of the user O to decrease by 0.3 to 0.9 dB in a frequency range of 1 Hz to 4 Hz.

In an embodiment, the wearable device 100 may control the stimulus unit so that the stimulus unit may provide second electrical stimulation that causes PSD of EEG of the user O to decrease by 0.2 to 0.5 dB in a frequency range of 4 Hz to 8 Hz.

In an embodiment, the wearable device 100 may control the stimulus unit so that the stimulus unit may provide third electrical stimulation that causes PSD of EEG of the user O to increase by 0.1 to 1.1 dB in a frequency range of 12 Hz to 25 Hz.

The wearable device 100 may further include a verification member that verifies a smoking effect. The verification member may measure a heart rate variability (HRV).

An HRV may refer to a comprehensive combination of indicators, such as a heart rate (HR), a mean interval between electrocardiogram (ECG) peaks (mean R-R interval), a standard deviation of normal to normal R-R intervals (SDNN), which indicates stress resistance (physiological resilience), a root mean square of successive heartbeat interval differences (RMSSD), which is a parasympathetic index that is involved in electrical stability of the heart, low frequency (LF), which is a relatively low-frequency component and an indicator of a sympathetic nervous activity, high frequency (HF), which is a relatively high-frequency component and related to a respiratory activity, and the like. A healthier person may have a complex HRV. Here, an increase in an HR may indicate an excitement and an increase in load (physical activity), a decrease in an SDNN may indicate a monotonous HR fluctuation signal, a decrease in an ability to cope with stress, and a decrease in overall health, a decrease in an RMSSD may indicate a heart abnormality, and an increase in an LF may indicate a decrease in an HRV.

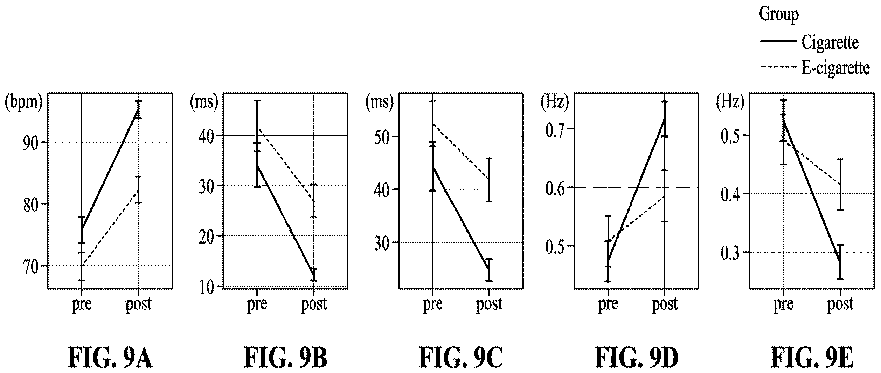

FIGS. 9A and 9B show the indicators of an HRV of a user before and after smoking a cigarette or an e-cigarette. In the case of an e-cigarette, effects of smoking an e-cigarette containing nicotine on ECG indicators are shown. FIG. 9A illustrates a mean heart rate (HR), FIG. 9B illustrates an RMSSD, FIG. 9C illustrates an SDNN, FIG. 9D illustrates an LF band, and FIG. 9E illustrates an HF band. Referring to FIGS. 9A to 9E, the mean HR and the LF band increased, while the SDNN, RMSSD, and the HF band decreased. This may indicate a physiological and psychological state similar to alertness in response to stimulation.

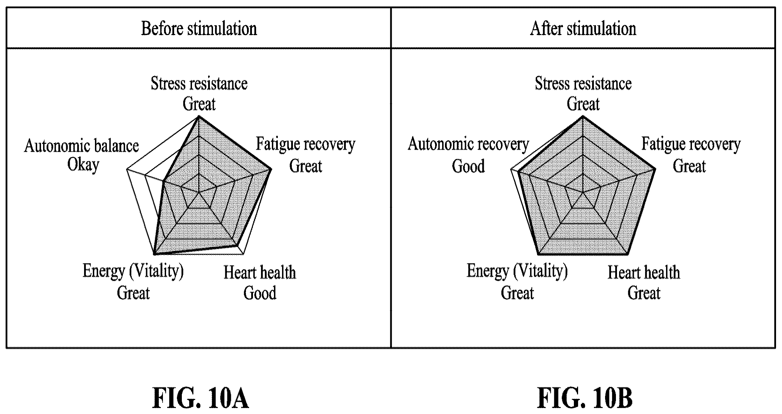

FIGS. 10A and 10B are diagrams illustrating a change in HRV indicators (e.g., an autonomic balance) caused by electrical stimulation through the wearable device 100. FIG. 10A illustrates the autonomic balance before the electrical stimulation and FIG. 10B illustrates the autonomic balance after the electrical stimulation. As shown in FIGS. 10A and 10B, it may be confirmed that the autonomic balance improves after the electrical stimulation compared to before the electrical stimulation. This may indicate a physiological and psychological state similar to alertness in response to stimulation.

The alertness effect after stimulation (providing a smoking effect) by the wearable device 100 may be confirmed through a verification member. For example, the verification member may utilize an SDNN or an RMSSD among the HRV indicators to confirm a decrease in the SDNN or the RMSSD and/or a degree of decrease in the SDNN and the RMSSD before and after the stimulation and may thus verify whether a sufficient alertness effect (i.e., a smoking effect) has been achieved.

The controller of the wearable device 100 may determine a degree of activation of the first stimulus unit 121, the second stimulus unit 122, and/or the second stimulus unit 123 based on a smoking indicator (e.g., the HRV indicators) signal transmitted from the verification member. For example, when a degree of decrease in the SDNN or the RMSSD confirmed by the verification member is sufficiently equivalent to a prestored degree of decrease in the SDNN or the RMSSD in actual smoking, the controller may maintain a control state of the first stimulus unit 121, the second stimulus unit 122, and/or the second stimulus unit 123. On the contrary, when a degree of decrease in the SDNN or the RMSSD confirmed by the verification member is not sufficiently equivalent to the prestored degree of decrease in the SDNN or the RMSSD in actual smoking, the controller may increase or decrease a stimulation intensity of the first stimulus unit 121, the second stimulus unit 122, and/or the second stimulus unit 123.

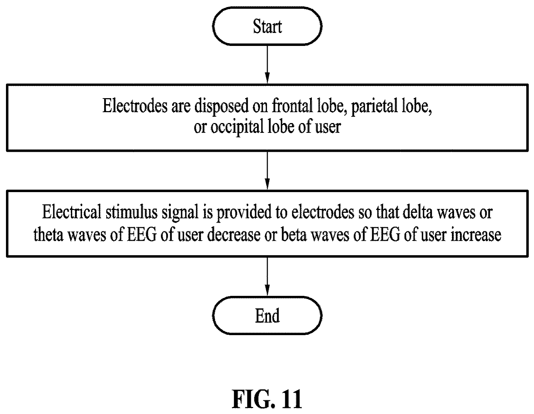

FIG. 11 is a diagram illustrating a method of providing a smoking effect, according to an embodiment.

Referring to FIG. 11, the method of providing a smoking effect may include an operation in which electrodes are disposed on at least two of the frontal lobe, the parietal lobe, and the occipital lobe of a user and an operation in which an electrical stimulus signal is provided to the electrodes so that delta waves or theta waves of EEG of the user may decrease or beta waves of the EEG of the user may increase.

In the operation in which an electrical stimulus signal is provided to the electrodes, an electrical stimulus signal may be provided to the electrodes disposed on positions corresponding to the parietal lobe or the occipital lobe, and accordingly, the delta waves may decrease.

In the operation in which an electrical stimulus signal is provided to the electrodes, an electrical stimulus signal may be provided to electrodes disposed on positions corresponding to the frontal lobe or the occipital lobe, and accordingly, the theta waves may decrease.

In the operation in which an electrical stimulus signal is provided to the electrodes, at least one of the frontal lobe, the parietal lobe, or the occipital lobe may be activated, and accordingly, the beta waves may increase.

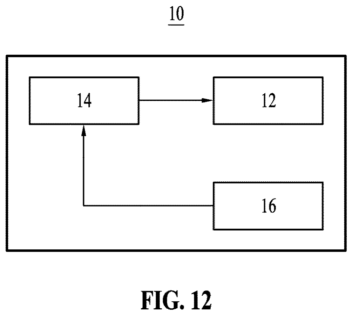

FIG. 12 is a block diagram illustrating a system 10 for providing a smoking effect, according to an embodiment.

Referring to FIG. 12, the system 10 for providing a smoking effect may include a stimulus unit 12 (e.g., the first stimulus unit 121, the second stimulus unit 122, or the third stimulus unit 123 of FIG. 2) including a stimulator that may perform brain stimulation on a brain of a user, and a controller 14 that may control a stimulation signal of the stimulus unit 12. The controller 14 may control the stimulus unit 12 so that the stimulus unit 12 may decrease at least one of delta waves or theta waves of EEG of the user or increase beta waves of EEG of the user.

In an embodiment, the stimulus unit 12 may provide electrical stimulation to a position corresponding to the dorsolateral prefrontal cortex of the user.

In an embodiment, the system 10 for providing a smoking effect may further include a verification member 16 including a verifier that may verify a smoking effect of the user, and the verification member 16 may transmit a smoking indicator signal detected from the user to the controller 14.

In an embodiment, the controller 14 determine a degree of activation of the stimulus unit 12 based on a smoking indicator (e.g., the HRV indicators) signal of the user transmitted from the verification member 16.

According to the wearable device 100 and the method of providing a smoking effect, a smoking effect may be provided to the user without an act of smoking. In this case, a smoking effect may effectively be provided through stimulation of the brain of the user. The wearable device 100 and the method of providing a smoking effect may maximize an alertness effect by varying regions to stimulate for each brain wave of the EEG of the user. As the wearable device 100 and a method for providing a smoking effect may adjust a degree of stimulus activation based on feedback (e.g., the HRV of the user) from the user, the degree of brain stimulation may effectively be controlled.

The descriptions of the above-described embodiments are only examples, and it will be understood by one of ordinary skill in the art that various changes and equivalents may be made therefrom. Therefore, the scope of the disclosure should be defined by the appended claims, and all differences within the scope equivalent to those described in the claims will be construed as being included in the scope of protection defined by the claims.

## Claims

1. A wearable device for providing a smoking effect, the wearable device comprising:
a housing;
a first stimulus unit provided on a surface of the housing and comprising at least one electrode that is disposed on a position corresponding to a first region of a user and configured to provide electrical stimulation;
a second stimulus unit provided on the surface of the housing and comprising at least one electrode that is disposed on a position corresponding to a second region of the user and configured to provide electrical stimulation; and
a controller configured to control the first stimulus unit or the second stimulus unit,
wherein the first stimulus unit or the second stimulus unit is configured to provide an electrical stimulus signal that induces an alertness state capable of implementing a smoking effect.

2. The wearable device of claim 1, further comprising:
a third stimulus unit provided on the surface of the housing,
wherein the third stimulus unit comprises at least one electrode that is disposed on a position corresponding to a third region of the user and configured to provide electrical stimulation.

3. The wearable device of claim 2, wherein
the first region is a frontal lobe,
the second region is a parietal lobe, and
the third region is an occipital lobe.

4. The wearable device of claim 3, wherein the controller is further configured to control at least one of the first stimulus unit, the second stimulus unit, or the third stimulus unit so that the first stimulus unit, the second stimulus unit, or the third stimulus unit provides a brain stimulus signal that decreases delta waves or theta waves of the user or increases beta waves of the user.

5. The wearable device of claim 3, wherein the controller is further configured to activate the second stimulus unit or the third stimulus unit to decrease delta waves of the user.

6. The wearable device of claim 3, wherein
the controller is further configured to activate the second stimulus unit or the third stimulus unit, and
the second stimulus unit or the third stimulus unit is configured to provide electrical stimulation that causes power spectral density (PSD) of electroencephalogram (EEG) of the user to decrease by 0.3 to 0.9 dB in a frequency range of 1 Hz to 4 Hz.

7. The wearable device of claim 3, wherein
the controller is further configured to activate the first stimulus unit or the third stimulus unit to decrease theta waves of the user.

8. The wearable device of claim 3, wherein
the controller is further configured to activate the first stimulus unit or the third stimulus unit, and
the first stimulus unit or the third stimulus unit is configured to provide electrical stimulation that causes PSD of EEG of the user to decrease by 0.2 to 0.5 dB in a frequency range of 4 Hz to 8 Hz.

9. The wearable device of claim 3, wherein the controller is further configured to activate at least one of the first stimulus unit, the second stimulus unit, or the third stimulus unit to increase beta waves of the user.

10. The wearable device of claim 3, wherein
the controller is further configured to activate at least one of the first stimulus unit, the second stimulus unit, or the third stimulus unit, and
at least one of the first stimulus unit, the second stimulus unit, or the third stimulus unit is configured to provide electrical stimulation that causes PSD of EEG of the user to increase by 0.1 to 1.1 dB in a frequency range of 12 Hz to 25 Hz.

11. The wearable device of claim 1, wherein the housing is configured to be worn on a head of the user.

12. A method of providing a smoking effect, the method comprising:
disposing electrodes on at least two of positions corresponding to a frontal lobe, a parietal lobe, and an occipital lobe of a user; and
providing an electrical stimulus signal to the electrodes so that delta waves or theta waves of electroencephalogram (EEG) of the user decrease or beta waves of the EEG of the user increase.

13. The method of claim 12, wherein, in the providing of the electrical stimulus signal to the electrodes, the electrical stimulus signal is provided to electrodes disposed on a position corresponding to the parietal lobe or the occipital lobe, and accordingly, the delta waves decrease.

14. The method of claim 12, wherein, in the providing of the electrical stimulus signal to the electrodes, the electrical stimulus signal is provided to electrodes disposed on a position corresponding to the frontal lobe or the occipital lobe, and accordingly, the theta waves decrease.

15. The method of claim 12, wherein, in the providing of the electrical stimulus signal to the electrodes, at least one of the frontal lobe, the parietal lobe, or the occipital lobe is activated, and accordingly, the beta waves increase.

16. A wearable device for providing a smoking effect, the wearable device comprising:
a housing;
a stimulus unit provided on a surface of the housing, configured to provide a stimulus signal, and comprising a stimulator; and
a controller provided in the housing and configured to control the stimulus unit,
wherein the controller is configured to control the stimulus unit so that the stimulus unit generates a stimulus signal that induces a state of alertness capable of implementing a smoking effect.

17. The wearable device of claim 16, wherein the stimulus unit is configured to perform at least one of electrical stimulation, acoustic stimulation, or optical stimulation, which stimulates a brain of a user.

18. The wearable device of claim 17, wherein the stimulus signal is transcranial alternating current stimulation (tACS), which decreases at least one of delta waves or theta waves of electroencephalogram (EEG) of the user or increases beta waves of EEG of the user.

19. The wearable device of claim 16, further comprising:
a verification member comprising a verifier configured to verify a smoking effect,
wherein the verification member is capable of measuring a heart rate variability (HRV).

20. The wearable device of claim 19, wherein the controller is configured to determine a degree of activation of the stimulus unit based on a smoking indicator signal transmitted from the verification member.
